(19) **Europäisches Patentamt / European Patent Office / Office européen des brevets**

(11) **EP 3 411 723 B1**

(12) # EUROPEAN PATENT SPECIFICATION

(45) Date of publication and mention
of the grant of the patent:
**11.10.2023 Bulletin 2023/41**

(21) Application number: **17705522.5**

(22) Date of filing: **31.01.2017**

(51) International Patent Classification (IPC):
**G01R 31/62** $^{(2020.01)}$    **H02H 7/04** $^{(2006.01)}$
**H01F 27/40** $^{(2006.01)}$    **G01N 33/28** $^{(2006.01)}$

(52) Cooperative Patent Classification (CPC):
**H02H 7/042; G01R 31/62; H01F 27/402;**
**H02H 5/041; H02H 5/06; H02H 5/086;**
G01N 33/2841

(86) International application number:
**PCT/US2017/015810**

(87) International publication number:
**WO 2017/146877 (31.08.2017 Gazette 2017/35)**

(54) **APPARATUS AND METHOD FOR MONITORING AND DIAGNOSING TRANSFORMER HEALTH**

VORRICHTUNG UND VERFAHREN ZUR ÜBERWACHUNG UND DIAGNOSE DER
TRANSFORMATORGESUNDHEIT

DISPOSITIF ET PROCÉDÉ DE SURVEILLANCE ET DE DIAGNOSTIC DE SANTÉ DE
TRANSFORMATEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.02.2016 IN 201644003795
05.01.2017 US 201715399392**

(43) Date of publication of application:
**12.12.2018 Bulletin 2018/50**

(73) Proprietor: **General Electric Company
Schenectady, NY 12345 (US)**

(72) Inventors:
• **PAMULAPARTHY, Balakrishna
Andhra
Pradesh 500 081 (IN)**
• **MUTHUKRISHNAN, Vijayasarathi
Markham, ON L6C 0M1 (CA)**
• **VINAYAGAM, Balamourougan
Markham, ON L6C 0M1 (CA)**
• **SEVOV, Lubomir
Markham, ON L6E 1B3 (CA)**

(74) Representative: **Brevalex
Tour Trinity
1 B Place de la Défense
92400 Courbevoie (FR)**

(56) References cited:
**WO-A1-2013/008246    US-A1- 2009 043 538
US-A1- 2009 091 867    US-A1- 2011 063 761**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

RELATED APPLICATION

The present application claims priority to and is a Continuation-In-Part of U.S. Patent Application No. 14/703,533, filed May 4, 2015, titled "INTEGRATED TRANSFORMER HEALTH MONITORING ARCHITECTURE", and further claims priority to Indian Patent Application No. 201644003795, filed February 3, 2016, and titled "SYSTEMS AND METHODS FOR MONITORING AND DIAGNOSING TRANSFORMER HEALTH".

FIELD OF THE DISCLOSURE

This disclosure relates to transformers, and more particularly, to an apparatus and a method for monitoring and diagnosing transformer health.

BACKGROUND OF THE DISCLOSURE

Various types of transformers are used in various types of applications. More particularly, power transformers are used ubiquitously in electrical power transmission systems to tailor voltage levels suitable for transmission over power lines. Such power transformers typically incorporate cooling mechanisms that are oil-based in order to dissipate heat generated in the transformer windings. However, over time, the chemical constituents of such oils tend to deteriorate and compromise transformer operations. For example, the viscosity of the oil in a power transformer may change over time and/or harmful gases may be released (for example as a result of a lightning strike) potentially leading to critical damage in the power transformer. Consequently, certain types of monitoring devices can be coupled to a power transformer in order to obtain gas samples from inside the transformer and to analyze these gas samples in order to evaluate the quality of the oil and to assess the health status of the power transformer.

The health status of the power transformer can be further monitored using other devices that detect various types of transformer faults and may take remedial action as well. For example, protection devices using sensors and relays may be used to monitor various currents and voltages associated with the power transformer and to isolate the power transformer from the power line for example, when a fault is detected in either the transformer itself or in the power line. One of the types of faults associated with a power transformer is known in the art as a through-fault. A typical through-fault can be characterized by a large amount of current propagating through the power transformer. When this current exceeds an operating current limit of the power transformer, the through-fault is deemed a major though-fault or a critical through-fault that necessitates immediate remedial action. For example, a short circuit in a load coupled to the power line, or a short circuit between the terminals of the power transformer, may necessitate immediate isolation of the power transformer from the power line. However, some other types of through-faults that occur in the power transformer can be more subtle in nature and comparatively harder to detect. For example, a current propagating through the power transformer may be below a trip limit of a protection device, yet significant enough to cause long-term damage to the power transformer if left unaddressed.

Consequently, certain types of transformer monitoring devices can be coupled to the power transformer in order to detect these types of subtle through-faults. These transformer monitoring devices can sense various types of transformer-related parameters (such as a primary winding current and a secondary winding current) in order to detect a through-fault based on small changes that can occur in one or more of these parameters. As can be understood, the amount of change in these various parameters can be very small and the effectiveness of various conventional transformer monitoring devices are often sub-optimal not only because they are unable to detect these small changes but also because of limitations in the algorithms and procedures used to process these subtle changes when detected.

Furthermore, conventional transformer monitoring devices are often configured to operate in an independent manner that focuses only on one primary function, such as detecting an electrical fault requiring immediate remedial action, detecting a through-fault requiring remedial action over a period of time, or analyzing a gas sample for detecting an oil-related issue in the transformer that is addressable over a period of time. Such conventional transformer monitoring devices fail to provide a holistic solution that addresses a combination of various factors that may interact with each other to adversely affect the health of a power transformer such as an oil-related gas buildup that may exacerbate a through-fault in the power transformer.

US2009/043538 discloses a diagnostic apparatus with an input interface for receiving data from a DGA apparatus, a processor configured to detect an abnormal gas-related condition and to generate transformer health data, and an output interface.

US2009/091867 discloses a device configured to receive monitor currents from the primary and secondary windings of a transformer and to detect a through-fault current.

WO2013/008246 discloses a transformer monitoring system, wherein a plurality of monitoring components is coupled to a data merging unit.

US2011/063761 discloses a method for transformer differential protection, including the calculation of differential and restraining currents.

BRIEF DESCRIPTION OF THE DISCLOSURE

The present invention provides an apparatus in accordance with claim 1, a system in accordance with claim 6, and a method in accordance with claim 13.

BRIEF DESCRIPTION OF THE DRAWINGS

Having thus described the disclosure in general terms, reference will now be made to the accompanying drawings, which are not necessarily drawn to scale, and wherein:

> FIG. 1 illustrates an example transformer health monitoring system that can include a diagnostic apparatus configured to monitor the health of a power transformer in accordance with an exemplary embodiment of the invention.
> FIG. 2 illustrates an exemplary diagnostic apparatus in accordance with an exemplary embodiment of the invention.
> FIG. 3 illustrates a chart that indicates an example relationship between a restraining current and a differential current flow in a transformer that is used to detect a through-fault-fault in accordance with an exemplary embodiment of the invention.
> FIG. 4 shows a flowchart of an example method of using a diagnostic apparatus to detect a through-fault in a transformer in accordance with an exemplary embodiment of the invention.

DETAILED DESCRIPTION OF THE DISCLOSURE

The disclosure will be described more fully hereinafter with reference to the accompanying drawings, in which exemplary embodiments of the disclosure are shown. This disclosure may, however, be embodied in many different forms and should not be construed as limited to the exemplary embodiments set forth herein; rather, these embodiments are provided so that this disclosure will satisfy applicable legal requirements. Like numbers refer to like elements throughout. It should be understood that certain words and terms are used herein solely for convenience and such words and terms should be interpreted as referring to various objects and actions that are generally understood in various forms and equivalencies by persons of ordinary skill in the art. For example, it should be understood that the word "line" as used herein generally refers to an electrical conductor, for example, a wire or an electrical power cable. The word "breaker" as used herein is not limited to a circuit breaker but generally refers to various types of protection elements that can be activated in order to protect a faulty transformer or equipment connected to a faulty transformer. The word "current" as used herein generally refers to an electrical current. Furthermore, the word "example" as used herein is intended to be non-exclusionary and nonlimiting in nature. More particularly, the word "exemplary" as used herein indicates one among several examples, and it should be understood that no undue emphasis or preference is being directed to the particular example being described.

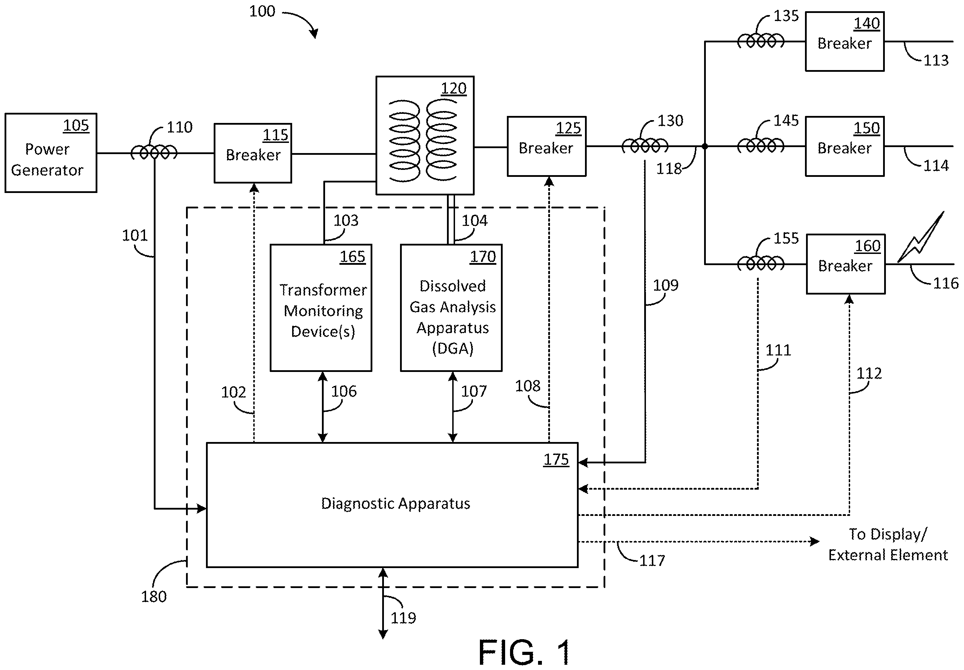

Attention is first drawn to FIG. 1, which illustrates an example transformer health monitoring system 100 including a diagnostic apparatus 175 configured to monitor the health of a power transformer 120. The power transformer 120 can be a single phase power transformer in one exemplary implementation and a multi-phase power transformer (for example, a three-phase power transformer) in another exemplary implementation. The input side and output side terminals (not shown) of the power transformer 120 can be connected to a single power line or multiple power lines (for example, three power lines) accordingly. However, solely for purposes of convenience, the power transformer 120 that is shown in FIG. 1 is a single-phase power transformer and various aspects will be described hereinafter in the context of a single-phase power transformer associated with a single-phase power system.

A power generator 105, which can generally refer to an electric power generating station or any other kind of power source, is coupled to the primary side of the power transformer 120 via a first current monitoring element 110 and a first breaker 115. The secondary side of the power transformer 120 is coupled to a line 118 via a second breaker 125 and a second current monitoring element 130. Furthermore the line 118 is coupled into three feeder lines 113, 114, and 116. Feeder line 113 includes a third current monitoring element 135 and a third breaker 140. Feeder line 114 includes a fourth current monitoring element 145 and a fourth breaker 150. Feeder line 116 includes a fifth current monitoring element 155 and a fifth breaker 160.

A transformer monitoring device 165 is coupled to the power transformer 120 via a line 103 for monitoring various transformer elements such as bushings and winding coils; various operating conditions such as temperature and moisture; and events such as switching events when a line is coupled or decoupled from a primary winding and/or a secondary winding of the power transformer 120. The exemplary transformer monitoring device 165, which can include one or more of a bushing monitor, a partial discharge monitor, and an on-load tap changer (OLTC) monitor, provides various types of monitoring data to the diagnostic apparatus 175 via one or more lines, such as a line 106. In some implementations, the line 106 can be a bi-directional communication link whereby the diagnostic apparatus 175 can not only receive monitoring data from the transformer monitoring device 165 but also transmit control signals to the transformer monitoring device 165 for executing various functions.

A dissolved gas analysis (DGA) apparatus 170 is coupled to the power transformer 120 via a suitable interface, such as a gas line 104, for monitoring and analyzing any gas buildup inside the power transformer 120. When gas is present in the power transformer 120 (for example as a result of a lightning strike on the power transformer 120 and/or on equipment associated with the power transformer 120) the DGA apparatus 170 executes one or more gas analysis procedures and provides DGA data to the diagnostic apparatus 175 via one or more lines, such as a line 107. The gas analysis procedures can include computing a DGA model, such as a Duval triangle. The DGA data can indicate to the diagnostic apparatus 175, an abnormal gas-related condition. In some implementations, the line 107 can be a bi-directional communication link whereby the diagnostic apparatus 175 can receive DGA data from the DGA apparatus 170, and, in some instances, also transmit control signals to the DGA apparatus 170 for executing various functions in the DGA apparatus 170.

In some exemplary implementations, the diagnostic apparatus 175, the transformer monitoring device 165, and the DGA apparatus 170 can be collectively located within an integrated enclosure 180. The integrated enclosure 180 can be a conventional enclosure that is used for housing conventional protection circuitry and as such, the diagnostic apparatus 175, the transformer monitoring device 165, and the DGA apparatus 170 can be used in some implementations to complement pre-existing conventional protection circuitry or to co-exist independently along with pre-existing conventional protection circuitry in a single enclosure.

The diagnostic apparatus 175 will be described below in further detail using other figures. However, in terms of a general overview, the diagnostic apparatus 175 is configured to generate health data of the power transformer 120 based on transformer-related information such as various voltages and currents present in the primary and secondary windings, equipment conditions (such as bushings and terminals), operating conditions (such as an abnormal temperature condition, and/or an abnormal OLTC operation), and DGA data. More particularly, in the exemplary embodiment shown in FIG. 1, the diagnostic apparatus 175 receives primary winding current information from the first current monitoring element 110 via a line 101, and secondary winding current information from the second current monitoring element 130 via a line 109. The diagnostic apparatus 175 further receives transformer operating conditions data from the transformer monitoring device 165 via the line 106, DGA data from the DGA apparatus 170 via the line 107, and electrical current information of one or more of the feeder line 113, the feeder line 114, and the feeder line 116 (via one or more lines that are generally referenced by the numeral 111).

The transformer health data generated in the diagnostic apparatus 175 can be transmitted by the diagnostic apparatus 175 in the form of one or more transformer health related signals and/or control signals to other elements such as a display unit (not shown) or an alarm unit (not shown) via a line 117, for example. Some examples of signals transmitted by the diagnostic apparatus 175 will be described below in further detail. The diagnostic apparatus 175 is also configured to allow a user (not shown) to communicate with the diagnostic apparatus 175 via a communications link 119 in order to provide the diagnostic apparatus 175 with various kinds of operating instructions and/or to access various types of information/data associated with the diagnostic apparatus 175.

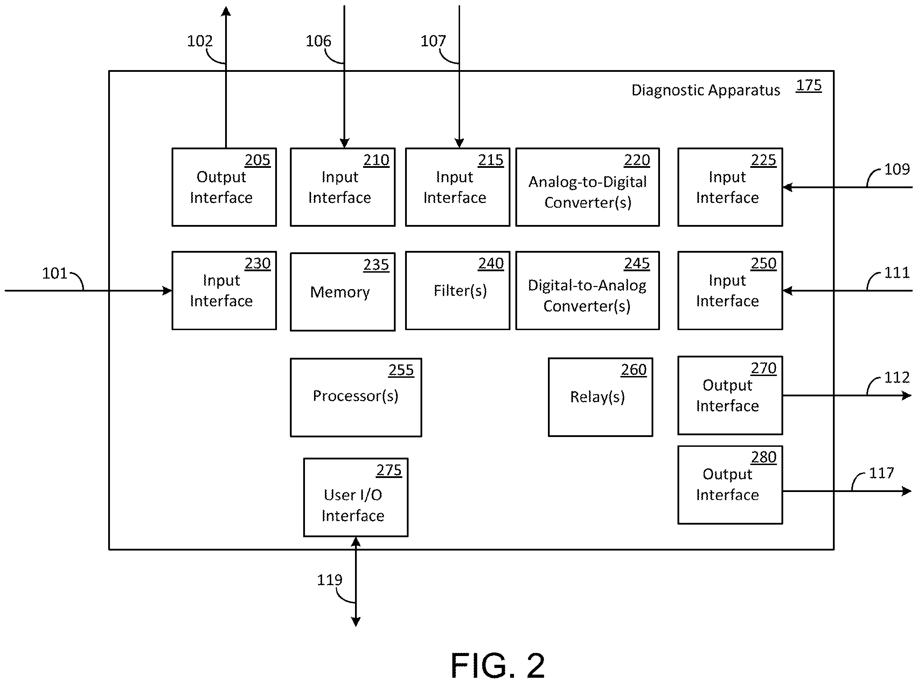

FIG. 2 illustrates some exemplary elements contained in the diagnostic apparatus 175. For purposes of description, the diagnostic apparatus 175 shown in FIG. 2 contains various elements for implementing the exemplary diagnostic apparatus 175 shown in FIG. 1 and described above with respect to the power transformer 120. Accordingly, the various input lines and output lines are designated by the same reference numerals that are shown in FIG. 1. However, in other implementations, such as, for example, when using the diagnostic apparatus 175 to monitor a three-phase transformer, the quantity and the nature of various elements contained (or used) in the diagnostic apparatus 175 can be different in order to match fewer or more input and output lines, for example.

The diagnostic apparatus 175 includes a number of input interfaces that are configured to receive various kinds of input information and a number of output interfaces that are configured to transmit various kinds of signals such as control signals and transformer health signals. A first input interface 230 is configured to receive primary winding current information via the line 101 coupled to the first current monitoring element 110. The line 101 can be a wire or a cable, for example, and can carry an electrical current in analog form. The electrical current carried on line 101 can be a scaled-down version of a primary current propagating through the primary winding of the power transformer 120. A second input interface 225 is configured to receive secondary winding current information via the line 109 from the second current monitoring element 130. The line 109 can be a wire or a cable, for example, that can carry an electrical current in analog form. The electrical current carried on line 109 can be a scaled-down version of the secondary current propagating through the secondary winding of the power transformer 120. A third input interface 215 is configured to receive DGA information from the DGA apparatus 170. The DGA information can be provided to the input interface 215 in the form of digital data over the line 107, which can be a digital communication link.

A fourth input interface 210 is configured to receive data from the transformer monitoring device 165. The data can be provided to the input interface 210 in the form of digital data over the line 106, which can be a digital communication link, for example. A fifth input interface 250 is configured to receive feeder current information via the line(s) 111 from one or more of the current monitoring elements 135, 145, and/or 155. The line(s) 111 can be a wire or a cable for example, that can carry an electrical current in analog form. The electrical current carried on line(s) 111 can be a scaled-down version of the current propagating through one or more of the feeder lines 113, 114, and 116.

The diagnostic apparatus 175 also includes one or more output interfaces such as a first output interface 205 that is shown coupled to the line 102 for purposes of transmitting a control signal to the breaker 115. Similarly, a second output interface (not shown) is coupled to the line 108 for purposes of transmitting a control signal to the breaker 125. A third output interface 270 is coupled to one or more lines (such as the line 112) for purposes of transmitting one or more control signal to one or more of the breakers 140, 150, and 160. A fourth output interface 280 is coupled to the line 117 for purposes of transmitting one or more transformer health related signals and/or control signals from the diagnostic apparatus 175 to an external element such as a display or an alarm unit for example.

The diagnostic apparatus 175 further includes one or more analog-to-digital converters and digital-to-analog converters. The analog-to-digital converter 220 is used to convert a current measurement provided by one of the input interfaces in an analog form into a digital current measurement value that can be processed by the processor 255. Conversely, the digital-to-analog converter 245 is used to convert various types of digital information that can be provided by the processor 255 to the digital-to-analog converter 245, into an analog output signal that can be transmitted out of the diagnostic apparatus 175 via the output interface 280, for example. One or more relays, such as a relay 260, are used for various types of switching purposes. For example, the relay 260 is used to switch various currents and/or alarm signals when a through-fault occurs in the power transformer 120.

One or more processors, such as the processor 255, are configured to communicatively cooperate with a memory 235. The processor 255 is implemented and operated using appropriate hardware, software, firmware, or combinations thereof. Software or firmware implementations include computer-executable or machine-executable instructions written in any suitable programming language to perform the various functions described. In one embodiment, instructions associated with a function block language are stored in the memory 235 and executed by the processor 255.

The memory 235 is used to store program instructions that are loadable and executable by the processor 255, as well as to store data generated during the execution of these programs. Depending on the configuration and type of the diagnostic apparatus 175, the memory 235 can be volatile (such as random access memory (RAM)) and/or non-volatile (such as read-only memory (ROM), flash memory, etc.). In some embodiments, the memory devices can also include additional removable storage (not shown) and/or non-removable storage (not shown) including, but not limited to, magnetic storage, optical disks, and/or tape storage. The disk drives and their associated computer-readable media can provide non-volatile storage of computer-readable instructions, data structures, program modules, and other data. In some implementations, the memory 235 can include multiple different types of memory, such as static random access memory (SRAM), dynamic random access memory (DRAM), or ROM.

The memory 235, the removable storage, and the non-removable storage are all examples of non-transient computer-readable storage media. Such non-transient computer-readable storage media can be implemented in any method or technology for storage of information such as computer-readable instructions, data structures, program modules or other data. Additional types of non-transient computer storage media that can be present include, but are not limited to, programmable random access memory (PRAM), SRAM, DRAM, ROM, electrically erasable programmable read-only memory (EEPROM), compact disc read-only memory (CD-ROM), digital versatile discs (DVD) or other optical storage, magnetic cassettes, magnetic tapes, magnetic disk storage or other magnetic storage devices, or any other medium which can be used to store the desired information and which can be accessed by the processor 255. Combinations of any of the above should also be included within the scope of non-transient computer-readable media.

Turning to the contents of the memory 235, the memory 235 can include, but is not limited to, an operating system (OS) and one or more application programs or services for implementing the features and aspects disclosed herein. Such applications or services include a transformer health data generation module (not shown). In one embodiment, the transformer health data generation module can be implemented by software that is provided in configurable control block language and is stored in non-volatile memory. When executed by the processor 255, the transformer health data generation module implements the various functionalities and features described in this disclosure.

A few operational and applications aspects of the diagnostic apparatus 175 will now be described in more detail. The diagnostic apparatus 175 is used to sample and/or collect electrical data associated with the power transformer 120 either at predetermined intervals or continuously (e.g., in real time). As a part of the sampling procedure, retrieved electrical data is assigned a time-stamp that corresponds to a moment in time at which the sampling of the electrical data occurred. After time-stamping, the electrical data is stored in the memory 235 or in an offsite server, a cloud server, and/or the like (not shown) and accessed by the processor 255 for analysis purposes.

The processor 255 analyzes the stored electrical data to determine if and when an event has occurred with respect to the power transformer 120. For example, the processor 255 analyzes the electrical data to identify a maximum or minimum parameter value (e.g., a voltage) of the power transformer 120 that exceeds a predetermined allowable threshold. In this way, the processor 255 recognizes or otherwise detects an event, such as an abnormality in the electrical data, which may correlate to an operational malfunctioning of the power transformer 120. An event may also include one or more of a variety of circumstances with respect to the power transformer 120, including, but not limited to, a power failure, a mechanical failure, manual or automatic trip, an electrical failure, an operational fault, a power leak, a triggered alarm, a parameter that meets and/or exceeds a predetermined threshold, and/or the like.

The processor 255 further generates a confidence score of the likelihood that an identified abnormality in electrical data is correlated to an operational failure of the power transformer 120. Furthermore, based at least in part on determining that an event has indeed occurred, the diagnostic apparatus 175 transmits a command to one or more monitoring devices, such as the transformer monitoring device 165 and/or the DGA apparatus 170, to initiate sampling data from the power transformer 120.

The one or more monitoring devices are used by the diagnostic apparatus 175 to retrieve and/or collect data (e.g., mechanical or electrical or signature data) pertaining to mechanical issues and/or health conditions of the power transformer 120. For example, the DGA apparatus 170 is configured to retrieve data through analyses of gas particles of oil in the power transformer 120. Example analyses of gas particles of oil can include, but are not limited to, photoacoustic spectroscopy and gas chromatography. Different types of DGA apparatuses may be utilized for retrieving different types of DGA data (e.g., based on particle size, oil type, and/or the like).

In some implementations, more than one DGA apparatus may be used. Consequently, more computer processing and more data processing time (e.g., more computing power and analysis may be required to acquire DGA data than acquiring electrical health data of the power transformer 120. In such applications, the processor 255 may use or determine a time-stamp offset to be added to a time-stamp of at least one of a previously time-stamped electrical data or a previously acquired DGA data. In this way, the diagnostic apparatus 175 can ensure that time-stamps of all received data (e.g., electrical data and DGA data) are accurately synchronized with respect to each other.

Furthermore, because obtaining DGA data from the power transformer 120 using more than one DGA apparatus can be a time-consuming process, a sampling routine can be scheduled in advance by the diagnostic apparatus 175. For example, the diagnostic apparatus 175 may determine whether any one of multiple DGA apparatuses is currently obtaining DGA data samples from the power transformer 120 (e.g., determine a state of the one or more DGA apparatus) by continuously (or intermittently) monitoring the state(s) of one or more of the DGA apparatuses. If it is determined that a DGA apparatus is currently obtaining DGA data from the power transformer 120 (for example, by determining that the DGA apparatus is not in a standby mode of operation), then the diagnostic apparatus 175 may allocate the DGA apparatus a next available time-stamp. On the other hand, if it is determined that the DGA apparatus is not currently obtaining DGA data from the power transformer 120 (for example, by determining that the DGA apparatus is in a standby mode of operation), then the diagnostic apparatus 175 may prompt the DGA apparatus on standby to begin sampling immediately or to begin sampling at a predetermined instant in time later on.

The processor 255 is further configured to recall and/or retrieve stored data (e.g., electrical data and DGA data) for one or more analyses. For example, in response to determining that an event has occurred with respect to the power transformer 120, the processor 255 recalls data that was time-stamped immediately prior to the time-stamp of the determined occurred event (e.g., electrical pre-data and DGA pre-data) and/or data that was time-stamped immediately after the time-stamp of the determined occurred event (e.g., electrical post-data and DGA post-data). Recalling pre-data and post-data includes determining a time-stamp of electrical data and DGA data stored in memory and identifying electrical data and DGA data stored in memory as electrical pre-data, electrical post-data, DGA pre-data, and/or DGA post-data based at least in part on their respective time-stamps.

The processor 255 analyzes pre-data and post-data for a variety of purposes. For example, pre-data and post-data may be analyzed to determine a cause of the event that occurred with respect to the power transformer 120 or understand the amount of stress experienced by power transformer 120 due to the event. In this way, pre-data and post-data can be utilized to understand the status of the power transformer 120 immediately prior to and immediately after the occurrence of the event, as pre-data and post-data may be indicative of particular extreme parameters or other factors that contributed to occurrence of the event (for example, a failure). The processor 255 may also determine, based at least in part on an analysis of data (e.g., electrical data, DGA data, pre-data, and/or post-data) an event type.

An analysis of data by the processor 255 includes may comparing recently received electrical data and/or DGA data to historical data (e.g., historical electrical data and/or historical DGA data) stored in memory. In this manner, the processor 255 is configured to compare recently received data to historical data that is indicative of an occurrence of a particular historical event with respect to the power transformer 120. Based at least in part on determining a match between the recently received data and historical data that is indicative of an occurrence of a historical event to the same power transformer 120 or another transformer, the processor 255 may determine that an event of the same event type as the historical event has recently occurred to the power transformer 120. A similar analysis may also be implemented by the processor 255 to determine an event type and/or event cause of a recently-occurred event.

Based at least in part on an analysis of data, the processor 255 is configured to determine and/or predict a cause of an event determined to have occurred with respect to the power transformer 120. In some embodiments, the processor 255 can be further configured to predict a future event, a cause of a future event, and/or a type of a future event, such as a failure, of the power transformer 120. In response to predicting a future event of the power transformer 120, the processor 255 may generate and/or schedule a maintenance request to repair the power transformer 120, log it as an event in database, and raise alarm/caution/warning prior to an occurrence of the predicted future event. The diagnostic apparatus 175 may transmit maintenance or frequent monitoring requests via the line 117 to a maintenance server and/or designated authorized person or persons.

In some embodiments, analyzing data can include determining one or more parameters of the power transformer 120 (for example, an electrical parameter, a DGA-related parameter and/or the like) no longer complies with one or more regulatory standards and/or fault codes. In order to do this, the processor 255 may compare received and/or historic data of the power transformer 120 to data associated with a transformer that does not comply with one or more regulatory standards and/or fault codes. Based at least in part on determining at least a partial match between the received and/or historic data of the power transformer 120 and data associated with a transformer that does not comply with one or more regulatory standards and/or fault codes, the processor 255 may determine that the power transformer 120 needs maintenance and/or replacement.

Additionally, the processor 255 can be configured to generate one or more reports based at least in part on an analysis of data. For example, the processor 255 may generate an energization record that highlights identified power and/or electrical issues of the power transformer 120 based at least in part on parameters associated with received electrical data such as a power failure, extreme fluctuations in current, and/or the like. Further DGA data captured during energization can be correlated with electrical data.

In another example, the processor 255 may generate a learned data record that includes actual received data of the power transformer 120 and a plot of the actual data. The processor 255 may, based at least in part on the analysis and/or plot of actual data, identify and/or predict one or more trends of transformer behavior.

In another embodiment, the processor 255 may generate a fault report that can include an analysis of stress levels incurred by the power transformer 120 prior to, during, and after occurrence of an event. A fault report may also include a determined change in parameter levels of the power transformer 120 between pre-data and post-data. The processor 255 may further determine a lifespan of the power transformer 120 based at least in part on an analysis of data and/or calculated data points such as a number of events (e.g., faults), their event types, a frequency of events, and/or the like incurred by the power transformer 120.

The processor 255 may also generate a historical max record. The processor 255 can be configured to compare each received data value (e.g., incoming electrical data and transformer health data) to a historical maximum value stored in memory of a similar data and/or parameter type. The processor 255 can determine which of the two data values is a maximum (or a minimum, an average, and/or the like), and can assign a corresponding maximum tag to the determined maximum. In this manner, the diagnostic apparatus 175 can keep track of (for example, by storing in the memory 235) extreme values for each parameter of the transformer along with a time stamp where so desired.

The processor 255 may also generate a transformer health report that can summarize an analysis of some or all data of the power transformer 120. The transformer health report may include some or all measured parameters of the power transformer 120 at each point in time, recommendations and/or status of maintenance and/or maintenance requests, and/or the like.

A transformer fleet report may also be generated by the processor 255. The transformer fleet report can include one or more transformer health reports for multiple transformers in a power system or utility grid. The transformer fleet report may also include a determined capacity of a power plant and/or geographic area of a power transmission & distribution system, identified events and/or problematic transformers, and/or the like.

Each of the reports and/or data itself may be transmitted to another computing device (not shown) for review, additional processing, and/or display. In some embodiments, data and/or reports may be presented to a user via a display on a mobile computing device and/or communication device. In other embodiments, data and/or reports may be generated and transmitted as a document, an email, a message, a tweet, a text, and/or the like. The data and/or reports may also be uploaded to a network-based server or a cloud based server so that they are accessible by one or more users. User authentication may be needed for accessing the data and/or reports.

The diagnostic apparatus 175 is further configured to utilize the processor 255 to control operation of the power transformer 120. For example, if the diagnostic apparatus 175 determines, based at least in part on an analysis of data of the power transformer 120, that the power transformer 120 has experienced an event that includes a failure of a mechanical component(s) of the power transformer 120, then the diagnostic apparatus 175 will (in response to determining a particular event type), shut off operation or otherwise control operation of the power transformer 120 (via operating one or more breakers, such as the breaker 115, the breaker 125, and/or the breaker 160). In this context, attention is drawn to FIG. 1, which shows a lightning strike having occurred on the feeder line 116, which may have led to a malfunction in the power transformer 120 that is detected by the DGA apparatus 170 and communicated to the diagnostic apparatus 175. The diagnostic apparatus 175 then activates the breaker 160 to isolate the feeder line 116.

The diagnostic apparatus 175 may also be coupled to one or more power transformers in addition to the power transformer 120. In this manner, the diagnostic apparatus 175 may enable the communication, transmittal, receipt, and/or sharing of data between transformers and/or transformer control systems. In some embodiments, the diagnostic apparatus 175 can be configured to monitor one or more transformers, analyze data associated with a plurality of transformers, and/or control one or more transformers.

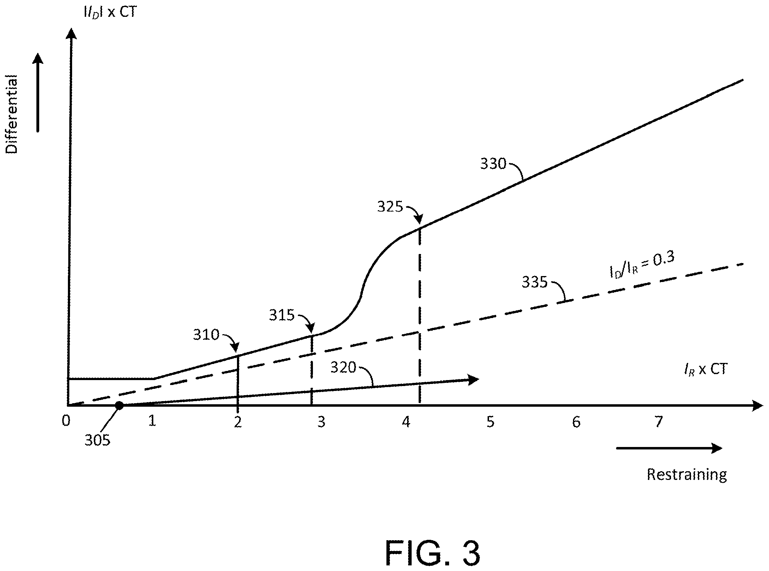

Attention is now drawn to FIG. 3, which shows a chart that indicates a relationship 330 between a restraining current and a differential current flow in the power transformer 120. The relationship 330 shown in the chart is used to detect a through-fault in accordance with the present invention. In a broad sense, a through-fault can be identified and declared when a small differential current (or no differential current) is detected to be co-existing with a high restraining current in the power transformer 120. Such an event can generally coincide with a saturation free time that occurs during the first few milliseconds after an inception of a fault in the power transformer 120.

The example chart shown in FIG. 3, graphically illustrates a trajectory 320 that resembles an example through-fault and current saturation in the power transformer 120. A directional principle is used to check if one or more currents with significant magnitudes (as compared with a fault current) flow in one direction (which is indicative of an internal fault in the power transformer 120) or one current flows in an opposite direction to a sum of other currents (which is indicative of a fault that is external to the power transformer 120). The diagnostic apparatus 175 executes a procedure that continuously calculates per-phase differential and restraining current values and compares a ratio between a per-phase differential and a restraining current value against a user pre-defined differential/restraining characteristic. Such a procedure is used to define a zone of differential protection operation and/or a zone of no operation and also to determine one or more set points relating to sensitivity, dependability, and security of the power transformer 120 under various types of faults. The set points are programmed by an operator of the diagnostic apparatus 175 to reflect various transformer differential protection applications.

The differential current value are calculated on a per-phase basis as a vector sum of currents from all winding of the power transformer 120 after magnitude and angle compensation is provided. This operation is defined by the following equation:

$$Id = \vec{I}_{1_{comp}} + \vec{I}_{2_{comp}} + \vec{I}_{3_{comp}}$$

The restraining current value is calculated as a maximum of internally compensated currents of the power transformer 120 and is defined by the following equation:

$$Ir = \max\left(\left|\vec{I}_{1_{comp}}\right|, \left|\vec{I}_{2_{comp}}\right|, \left|\vec{I}_{3_{comp}}\right|\right)$$

An exemplary condition for declaring a through-fault detection condition based on percentage differential protection can be defined by the following equations:

$$Id/Ir < 0.3 \ \& \ Ir > 2x \ CT,$$

where CT represents at least one of a primary winding current value derived from the electrical current measurement obtained via the first current monitoring element 110 or a secondary winding current value derived from the electrical current measurement obtained via the second current monitoring element 130.

The chart shown in FIG. 3 can be better described with respect to the example transformer health monitoring system 100 shown in FIG. 1. During normal operation of the power transformer 120, a differential current value calculated by the diagnostic apparatus 175 using the current measurements provided via the lines 101 and 109 has a low amplitude. However, when an external fault occurs (such as a lightning strike on the feeder line 116) the differential current value has a significantly higher amplitude. The differential current value as well as other measurements, in particular a ratio between the differential current value and a restraining current value, are used by the diagnostic apparatus 175 for defining operate regions and block regions of one or more breakers, such as the breakers 115, 125, 140, 150, and 160.

Under normal loading conditions of the power transformer 120, the differential current value is substantially zero (as indicated by the dot 305). The restraining current value corresponding to the dot 305 is typically set to less than a current rating of the power transformer 120. When an external fault occurs, the trajectory 320 of the Id/Ir ratio always goes towards the right of an initial normal loading condition. Furthermore, in one example implementation, when the trajectory 320 indicates an Id/Ir ratio that is less than 0.3 x CT rating and the restraining current is higher than 2 x CT rating of the power transformer 120, a transformer through-fault flag 310 can be generated. In other example implementations, other values of the Id/Ir ratio and the restraining current can be used for generating the through-fault flag 310. For example, in lieu of the integers 0.3 and 2 that are used in the 0.3 x CT rating and the 2 x CT rating described above with reference to the first example implementation, other integers can be used. These other integers can be based on various sensitivity characteristics associated with a through-fault.

When the through-fault flag 310 is generated, the diagnostic apparatus 175 can start obtaining pre-trigger and post-trigger data pertaining to the power transformer 120 as described above. The region of the trajectory 320 that is located between break-points 315 and 325 is indicative of a saturation-free period of operation of the power transformer 120.

Following the through-fault detection procedure described above with reference to FIGs 1 and 3, the diagnostic apparatus 175 can execute various types of analysis procedures on not just the power transformer 120 but on associated components as well. Such associated components can include bushings and other fixtures for example. The analysis procedures can generally encompass monitoring of various thermal parameters, insulation parameters, bushing parameters, DGA parameters, load tap changes (LTCs), and protection integrity checks associated with one or more breakers.

Having described various features and actions associated with the exemplary transformer health monitoring system 100, and particularly the diagnostic apparatus 175, a general overview of various additional aspects in accordance with the disclosure will now be provided below.

In an example implementation, the diagnostic apparatus 175 can also be communicatively coupled to suitable oscillography apparatus for executing various types of oscillography procedures. For example, the diagnostic apparatus 175 can transmit a trigger signal to an oscillography apparatus upon detecting a through-fault in the power transformer 120. Oscillography data can then be captured at a "n" samples per cycle sampling rate where "n" is any suitable integer that is selected in order to capture data over "m" integer number of cycles. For example, a sampling rate of 50 samples per cycle or 60 samples per cycle can be used in some implementations.

Using such captured data from oscillography procedures, as well as other procedures, a statistical analysis of pre-fault and post-fault conditions can be carried out by the diagnostic apparatus 175. The statistical analysis can include calculation of various values pertaining to variance and deviations and comparison against threshold values.

In another example implementation, the diagnostic apparatus 175 can be configured to capture data associated with one or more bushings based on a sampling procedure for example, upon detection of a through-fault. The sampling procedure can include collection of pre-fault and post-fault data from the one or more bushings.

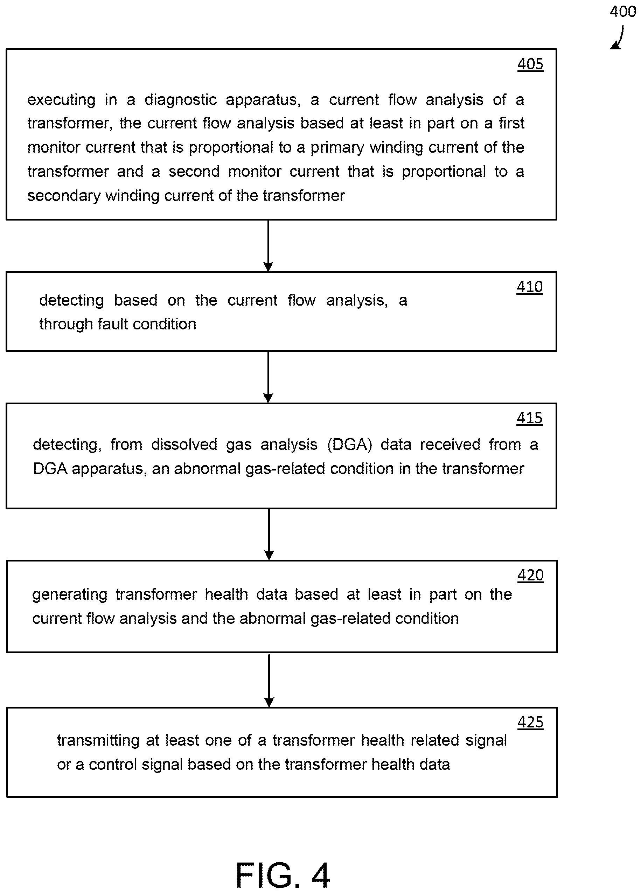

FIG. 4 shows a flowchart of an example method of using the diagnostic apparatus 175 to detect a through-fault in the power transformer 120, in accordance with an exemplary embodiment of the invention. In block 405, an electrical current flow analysis of the power transformer 120 is executed by the diagnostic apparatus 175. The electrical current flow analysis based at least in part on a first monitor current that is proportional to a primary winding current of the transformer and a second monitor current that is proportional to a secondary winding current of the transformer. As shown in FIG. 1, the first monitor current can be provided to the diagnostic apparatus 175 from the first current monitoring element 110 via the line 101, and the second monitor current can be provided to the diagnostic apparatus 175 from the second current monitoring element 130 via the line 109. In block 410, a through-fault condition is detected based on the current flow analysis. In block 415, an abnormal gas-related condition in the power transformer 120 is detected by the diagnostic apparatus 175 using DGA data provided by the DGA apparatus 170. In block 420, transformer health data is generated based at least on the current flow analysis and the abnormal gas-related condition. In block 425, a transformer health related signal and/or a control signal is transmitted by the diagnostic apparatus 175 based on the transformer health data.

Many modifications and other embodiments of the example descriptions set forth herein to which these descriptions pertain will come to mind having the benefit of the teachings presented in the foregoing descriptions and the associated drawings. Thus, it will be appreciated the disclosure may be embodied in many forms and should not be limited to the exemplary embodiments described above. Therefore, it is to be understood that the disclosure is not to be limited to the specific embodiments disclosed and that modifications and other embodiments are intended to be included within the scope of the appended claims. Although specific terms are employed herein, they are used in a generic and descriptive sense only and not for purposes of limitation.

**Claims**

1. A diagnostic apparatus (175) comprising:

a first input interface (230) configured to receive a first monitor current that is proportional to a primary winding current of a transformer (120); a second input interface (225) configured to receive a second monitor current that is proportional to a secondary winding current of the transformer; and **characterized by** a third input interface (215) configured to receive DGA data from a dissolved gas analysis (DGA) apparatus (170); at least one processor (255) configured to:

- execute an electrical current flow analysis of the transformer based at least in part on the first monitor current and the second monitor current;
- detect, based on the electrical current flow analysis, a through-fault condition;
- detect from the received DGA data, an abnormal gas-related condition; and
- generate transformer health data based at least in part on the through-fault condition and the abnormal gas-related condition; and

an output interface configured to transmit from the diagnostic apparatus, at least one of a transformer health related signal or a control signal based on the transformer health data;
wherein the current flow analysis comprises:

calculating a differential electrical current value and a restraining electrical current value based on the primary winding current and the secondary winding current of the transformer;
deriving real-time electrical current flow data of the transformer by comparing a ratio of the differential electrical current value and the restraining electrical current value to a threshold value;
identifying a first instant in time at which the ratio exceeds the threshold value;
fetching from a storage element (235), historical electrical current flow data of the transformer;
generating cumulative electrical current flow data of the transformer by combining the historical electrical current flow data and real-time electrical current flow data obtained at the first instant in time and subsequent to the first instant in time; and
detecting the through-fault condition based on the cumulative electrical current flow data of the transformer.

2. The diagnostic apparatus of claim 1, further comprising:
a protection relay (260) configured to receive the control signal and modify or eliminate electrical current flow through at least one of a primary winding of the transformer or a secondary winding of the transformer by activating one or more protection elements coupled to the transformer.

3. The diagnostic apparatus of claim 1, wherein at least the first input interface (230), the second input interface (225), the third input interface (215), the output interface, the at least one processor (255), and the storage element (235) are collectively housed in an enclosure (180).

4. The diagnostic apparatus of claim 3, wherein the enclosure includes at least one of a communications port or a user interface (275) that allows a user to set the first threshold value.

5. The diagnostic apparatus of claim 1, wherein the first monitor current is provided to the first input interface by a first electrical current transformer coupled in series with a primary winding of the transformer (120) and the second monitor current is provided to the second input interface by a second electrical current transformer coupled in series with a secondary winding of the transformer (120).

6. A transformer health monitoring system (100) comprising:

at least one electrical current sensing element (165) coupled to a transformer (120);
a first breaker (115, 125) coupled to one of a primary winding of the transformer or a secondary winding of the transformer;
a dissolved gas analysis (DGA) apparatus (170) coupled to the transformer; and
a diagnostic apparatus (175) according to claim 1 coupled to the at least one electrical current sensing element and the DGA apparatus (170), and further coupled to the first breaker, wherein the diagnostic apparatus (175) is configured to transmit a control signal to activate the first breaker.

7. The system of claim 6, wherein the at least one electrical current sensing element comprises a first electrical current sensing element and a second electrical current sensing element, the first electrical current sensing element configured to sense a primary winding current of the transformer and the second electrical current sensing element configured to sense a secondary winding current of the transformer, and wherein the electrical current flow analysis comprises:

calculating a differential electrical current value and a restraining electrical current value based on the primary winding current and the secondary winding current of the transformer;
comparing a ratio of the differential electrical current value and the restraining electrical current value to a threshold value; and
declaring the through-fault condition when the ratio exceeds the threshold value.

8. The system of claim 6, further comprising at least one of a bushing monitor, a partial discharge monitor, or an on-load tap changer (OLTC) monitor coupled to the diagnostic apparatus (175), the diagnostic apparatus further configured to generate transformer health data based at least in part on event data received from the at least one of the bushing monitor, the partial discharge monitor, or the OLTC monitor.

9. The system of claim 6, wherein the control signal is configured to at least one of activate a first breaker coupled to one of a primary winding of the transformer or a secondary winding of the transformer, or to activate a second breaker for disconnecting at least a first feeder when a fault condition occurs in the first feeder, the first feeder being one of a plurality of feeders coupled to a secondary winding of the transformer.

10. The system of claim 6, further comprising a plurality of feeders (113, 114, 116) coupled to the secondary winding of the transformer, and further comprising a second breaker (160), wherein the diagnostic apparatus (175) is configured to transmit a control signal to activate the second breaker for disconnecting a first feeder (116) when at least a second feeder among the plurality of feeders is in a non-fault condition when a through-fault condition occurs in the first feeder (116).

11. The system of claim 6, wherein the transformer health related signal is at least one of an out-of-specification warning signal that indicates that the transformer no longer complies with one or more regulatory standards, a degradation warning signal indicative of a reduced lifespan of the transformer, or an early warning signal indicative of at least one of a cause or a characteristic of a future failure of the transformer.

12. The system of claim 6, wherein the diagnostic apparatus (175) is further configured to:

receive event data from a protection relay (260); generate a record based at least in part on the event data received from the protection relay (260) and the DGA data received from the DGA apparatus (170); and output, at least a portion of the record, via at least one of a user interface (275) or a communication link of the diagnostic apparatus (175).

13. A method comprising:

executing in a diagnostic apparatus (175) according to claim 1, an electrical current flow of a transformer (120), based at least in part on a first monitor current that is proportional to a primary winding current of the transformer and a second monitor current that is proportional to a secondary winding current of the transformer; detecting, based on the electrical current flow analysis, a through-fault condition; detecting, from dissolved gas analysis (DGA) data received from a DGA apparatus (170), an abnormal gas-related condition in the transformer; generating transformer health data based at least in part on the through-fault condition and the abnormal gas-related condition; and transmitting at least one of a transformer health related signal or a control signal based on the transformer health data; wherein executing the electrical current flow analysis comprises:

calculating a differential electrical current value and a restraining electrical current value based on the primary winding current and the secondary winding current of the transformer; deriving real-time electrical current flow data of the transformer by comparing a ratio of the differential electrical current value and the restraining electrical current value to a threshold value; identifying a first instant in time at which the ratio exceeds the threshold value; fetching from a storage element (235), historical electrical current flow data of the transformer; and generating cumulative electrical current flow data of the transformer by combining the historical electrical current flow data and real-time electrical current flow data obtained at the first instant in time and subsequent to the first instant in time; and detecting the through-fault condition based on the cumulative electrical current flow data of the transformer.

14. The method of claim 13, wherein the historical electrical current flow data is time-stamped, and wherein fetching the historical electrical current flow data is based at least in part on a definable period of time prior to the first instant in time.

15. The method of claim 14, further comprising: receiving in the diagnostic apparatus (175), a user input comprising at least one of the threshold value or a time period specifying the definable period of time.

16. The method of claim 13, further comprising: displaying on a display of the diagnostic apparatus (175), at least one of a transformer health status, the first instant in time at which the ratio exceeded the threshold value, or at least a portion of the cumulative electrical current flow data.

17. The method of claim 13, wherein DGA data received from the DGA apparatus (170) comprises at least one of DGA data collected by the DGA apparatus prior to a first instant in time or DGA data collected by the DGA apparatus after the first instant in time.

**18.** The method of claim 17, wherein the DGA data collected by the DGA apparatus (170) after the first instant in time is indicative of the transformer having at least one of a reduced performance, a compromised performance, a reduced lifespan, or an upcoming catastrophic failure.

**Patentansprüche**

**1.** Diagnostisches Gerät (175), umfassend:

Eine erste Eingabe-Schnittstelle (230), die so konfiguriert ist, dass sie einen ersten Überwachungsstrom empfängt, der proportional zu einem Primärwicklungsstrom eines Transformators (120) ist;
eine zweite Eingabe-Schnittstelle (225), die so konfiguriert ist, dass sie einen zweiten Überwachungsstrom empfängt, der proportional zu einem Sekundärwicklungsstrom des Transformators ist;
und **gekennzeichnet durch**
eine dritte Eingabe-Schnittstelle (215), die so konfiguriert ist, dass sie DGA-Daten von einem Gerät (170) zur Analyse gelöster Gase (DGA) empfängt;
mindestens einen Prozessor (255), der konfiguriert ist zum:

- Ausführen einer Analyse des elektrischen Stromflusses des Transformators, die mindestens teilweise auf dem ersten Überwachungsstrom und dem zweiten Überwachungsstrom basiert;
- Feststellen, basierend auf der Analyse des elektrischen Stromflusses, eines Zustands mit Durchgangsfehlern;
- Feststellen eines abnormalen gasbezogenen Zustands anhand der empfangenen DGA-Daten; und
- Erzeugen von Transformatorzustandsdaten, die mindestens teilweise auf dem Zustand des Durchgangsfehlers und dem abnormalen gasbezogenen Zustand basieren; und

eine Ausgabe-Schnittstelle, die so konfiguriert ist, dass sie von dem diagnostischen Gerät mindestens ein auf den Zustand des Transformators bezogenes Signal oder ein auf den Transformatorzustandsdaten basierendes Steuersignal sendet;
wobei die Analyse des Stromflusses Folgendes umfasst:

Berechnen eines elektrischen Differenzstromwertes und eines elektrischen Begrenzungsstromwertes basierend auf dem Primärwicklungsstrom und dem Sekundärwicklungsstrom des Transformators;
Ableiten von Daten über den Stromfluss des Transformators in Echtzeit durch Vergleich eines Verhältnisses zwischen dem elektrischen Differenzstromwert und dem elektrischen Haltestromwert mit einem Schwellenwert;
Identifizieren eines ersten Zeitpunkts, zu dem das Verhältnis den Schwellenwert überschreitet;
Abrufen von historischen Daten über den Stromfluss des Transformators aus einem Speicherelement (235);
Erzeugen kumulativer Stromflussdaten des Transformators durch Kombinieren der historischen Stromflussdaten und der Echtzeit-Stromflussdaten, die zum ersten Zeitpunkt und nach dem ersten Zeitpunkt ermittelt wurden; und
Feststellen des Zustands des Durchgangsfehlers basierend auf den kumulierten elektrischen Stromflussdaten des Transformators.

**2.** Diagnostisches Gerät nach Anspruch 1, ferner umfassend:
Ein Schutzrelais (260), das so konfiguriert ist, dass es das Steuersignal empfängt und den elektrischen Stromfluss durch mindestens eine Primärwicklung des Transformators oder eine Sekundärwicklung des Transformators modifiziert oder eliminiert, indem es ein oder mehrere mit dem Transformator gekoppelte Schutzelemente aktiviert.

**3.** Diagnostisches Gerät nach Anspruch 1, wobei mindestens die erste Eingabe-Schnittstelle (230), die zweite Eingabe-Schnittstelle (225), die dritte Eingabe-Schnittstelle (215), die Ausgabe-Schnittstelle, der mindestens eine Prozessor (255) und das Speicherelement (235) gemeinsam in einem Gehäuse (180) untergebracht sind.

**4.** Diagnostisches Gerät nach Anspruch 3, wobei das Gehäuse mindestens eine Kommunikationsschnittstelle oder eine Benutzerschnittstelle (275) einschließt, die es einem Benutzer ermöglicht, den ersten Schwellenwert einzustellen.

**5.** Diagnostisches Gerät nach Anspruch 1, wobei der erste Überwachungsstrom der ersten Eingabe-Schnittstelle durch einen ersten elektrischen Stromwandler bereitgestellt wird, der in Reihe mit einer Primärwicklung des Transformators (120) gekoppelt ist, und der zweite Überwachungsstrom der zweiten Eingabe-Schnittstelle durch einen zweiten elektrischen Stromwandler bereitgestellt wird, der in Reihe mit einer Sekundärwicklung des Transformators (120) gekoppelt ist.

6. Zustandsüberwachungssystem für Transformatoren (100), umfassend:

Mindestens ein elektrisches Stromerfassungselement (165), das mit einem Transformator (120) gekoppelt ist;
einen ersten Unterbrecher (115, 125), der entweder mit einer Primärwicklung des Transformators oder einer Sekundärwicklung des Transformators gekoppelt ist;
ein Gerät (170) zur Analyse gelöster Gase (DGA), das mit dem Transformator gekoppelt ist; und
ein diagnostisches Gerät (175) nach Anspruch 1, das mit dem mindestens einen elektrischen Stromerfassungselement und dem DGA-Gerät (170) gekoppelt ist und ferner mit dem ersten Unterbrecher gekoppelt ist, wobei das diagnostische Gerät (175) so konfiguriert ist, dass es ein Steuersignal zur Aktivierung des ersten Unterbrechers sendet.

7. System nach Anspruch 6, wobei das mindestens eine elektrische Stromerfassungselement ein erstes elektrisches Stromerfassungselement und ein zweites elektrisches Stromerfassungselement umfasst, wobei das erste elektrische Stromerfassungselement so konfiguriert ist, dass es einen Primärwicklungsstrom des Transformators erfasst und das zweite elektrische Stromerfassungselement so konfiguriert ist, dass es einen Sekundärwicklungsstrom des Transformators erfasst, und wobei die Analyse des elektrischen Stromflusses Folgendes umfasst:

Berechnen eines elektrischen Differenzstromwertes und eines elektrischen Begrenzungsstromwertes basierend auf dem Primärwicklungsstrom und dem Sekundärwicklungsstrom des Transformators;
Vergleichen eines Verhältnisses zwischen dem elektrischen Differenzstromwert und dem elektrischen Haltestromwert mit einem Schwellenwert; und
Erklären des Zustands des Durchgangsfehlers, wenn das Verhältnis den Schwellenwert überschreitet.

8. System nach Anspruch 6, ferner umfassend mindestens einer von einer Buchsenüberwachungen, einer Teilentladungsüberwachung, oder einer mit dem diagnostischen Gerät (175) gekoppelten Laststufenschalterüberwachung (OLTC), wobei das diagnostische Gerät ferner so konfiguriert ist, dass es Transformatorzustandsdaten erstellt, die mindestens teilweise auf Ereignisdaten basieren, die von der mindestens einen von der Buchsenüberwachung, der Teilentladungsüberwachung oder der OLTC-Überwachung empfangen werden.

9. System nach Anspruch 6, wobei das Steuersignal so konfiguriert ist, dass es mindestens eines von einem ersten Unterbrecher, der mit einer von einer Primärwicklung des Transformators oder einer Sekundärwicklung des Transformators gekoppelt ist, oder einem zweiten Unterbrecher aktiviert, um mindestens einen ersten Abzweig zu trennen, wenn ein Fehlerzustand in dem ersten Abzweig auftritt, wobei der erste Abzweig einer aus einer Vielzahl von Abzweigen ist, die mit einer Sekundärwicklung des Transformators gekoppelt sind.

10. System nach Anspruch 6, ferner umfassend eine Vielzahl von Abzweigern (113, 114, 116), die mit der Sekundärwicklung des Transformators gekoppelt sind, und ferner umfassend einen zweiten Unterbrecher (160), wobei das diagnostische Gerät (175) so konfiguriert ist, dass es ein Steuersignal sendet, um den zweiten Unterbrecher zum Trennen eines ersten Abzweigs (116) zu aktivieren, wenn mindestens ein zweiter Abzweig aus der Vielzahl von Abzweigen in einem fehlerfreien Zustand ist, wenn ein Zustand mit Durchgangsfehlern im ersten Abzweig (116) auftritt.

11. System nach Anspruch 6, wobei das auf den Zustand des Transformators bezogene Signal mindestens ein Warnsignal ist, das anzeigt, dass der Transformator nicht mehr mit einer oder mehreren Normen übereinstimmt, ein Degradationswarnsignal, das auf eine verkürzte Lebensdauer des Transformators hinweist, oder ein Frühwarnsignal, das auf mindestens eine Ursache oder eine Eigenschaft eines zukünftigen Ausfalls des Transformators hinweist.

12. System nach Anspruch 6, wobei das diagnostische Gerät (175) ferner konfiguriert ist zum:

Empfangen von Ereignisdaten von einem Schutzrelais (260);
Erzeugen einer Aufzeichnung, die mindestens teilweise auf den vom Schutzrelais (260) empfangenen Ereignisdaten und den vom DGA-Gerät (170) empfangenen DGA-Daten basiert; und
Ausgeben mindestens eines Abschnitts des Datensatzes über mindestens eine Benutzerschnittstelle (275) oder eine Kommunikationsverbindung des diagnostischen Geräts (175).

13. Verfahren, umfassend:

Ausführen in einem diagnostischen Gerät (175) nach Anspruch 1 eines elektrischen Stromflusses eines Transformators (120), mindestens teilweise basierend auf einem ersten Überwachungsstrom, der proportional zu einem Primärwicklungsstrom des Transformators ist, und einem zweiten Überwachungsstrom, der proportional zu einem Sekundärwicklungsstrom des Transformators ist;

Feststellen, basierend auf der Analyse des elektrischen Stromflusses, eines Zustands mit Durchgangsfehlern;

Feststellen eines anormalen gasbezogenen Zustands im Transformator anhand von Daten der Analyse gelöster Gase (DGA), die von einem DGA-Gerät (170) empfangen werden;

Erzeugen von Transformatorzustandsdaten, die mindestens teilweise auf dem Zustand des Durchgangsfehlers und dem abnormalen gasbezogenen Zustand basieren; und

Senden mindestens eines von einem mit dem Transformatorzustand verbundenen Signal oder einem Steuersignal basierend auf den Transformatorzustandsdaten ;

wobei die Durchführung der Analyse des elektrischen Stromflusses Folgendes umfasst:

> Berechnen eines elektrischen Differenzstromwertes und eines begrenzenden elektrischen Stromwertes basierend auf dem primären Wicklungsstrom und dem sekundären Wicklungsstrom des Transformators;
>
> Ableiten von elektrischen Stromflussdaten des Transformators in Echtzeit durch Vergleich eines Verhältnisses zwischen dem elektrischen Differenzstromwert und dem elektrischen Ruhestromwert mit einem Schwellenwert;
>
> Identifizieren eines ersten Zeitpunkts, zu dem das Verhältnis den Schwellenwert überschreitet;
>
> Abrufen von historischen elektrischen Stromflussdaten des Transformators von einem Speicherelement (235); und
>
> Erzeugen kumulativer Stromflussdaten des Transformators durch Kombinieren der historischen Stromflussdaten und der Echtzeit-Stromflussdaten, die zum ersten Zeitpunkt und nach dem ersten Zeitpunkt ermittelt wurden; und
>
> Feststellen des Zustands des Durchgangsfehlers basierend auf den kumulierten elektrischen Stromflussdaten des Transformators.

14. Verfahren nach Anspruch 13, wobei die historischen elektrischen Stromflussdaten mit einem Zeitstempel versehen sind, und wobei das Abrufen der historischen elektrischen Stromflussdaten mindestens teilweise auf einer definierbaren Zeitdauer vor dem ersten Zeitpunkt basiert.

15. Verfahren nach Anspruch 14, ferner umfassend: Empfangen einer Benutzereingabe in dem diagnostischen Gerät (175), die mindestens den Schwellenwert oder eine Zeitdauer umfasst, die die definierbare Zeitdauer angibt.

16. Verfahren nach Anspruch 13, ferner umfassend: Anzeigen auf einer Anzeige des diagnostischen Geräts (175) mindestens eines von einem Transformatorzustand, dem ersten Zeitpunkt, zu dem das Verhältnis den Schwellenwert überschritten hat, oder mindestens einem Abschnitt der kumulierten elektrischen Stromflussdaten.

17. Verfahren nach Anspruch 13, wobei die von dem DGA-Gerät (170) empfangenen DGA-Daten mindestens DGA-Daten umfassen, die von dem DGA-Gerät vor einem ersten Zeitpunkt oder DGA-Daten, die von dem DGA-Gerät nach dem ersten Zeitpunkt gesammelt wurden.

18. Verfahren nach Anspruch 17, wobei die von dem DGA-Gerät (170) nach dem ersten Zeitpunkt gesammelten DGA-Daten darauf hinweisen, dass der Transformator mindestens eines von einer reduzierten Leistung, einer beeinträchtigten Leistung, einer reduzierten Lebensdauer oder einem bevorstehenden katastrophalen Ausfall aufweist.

**Revendications**

1. Appareil de diagnostic (175) comprenant :

une première interface d'entrée (230) configurée pour recevoir un premier courant de surveillance qui est proportionnel à un courant d'enroulement primaire d'un transformateur (120) ;
une deuxième interface d'entrée (225) configurée pour recevoir un deuxième courant de surveillance qui est proportionnel à un courant d'enroulement secondaire du transformateur ;
et **caractérisé par**
une troisième interface d'entrée (215) configurée pour recevoir des données DGA en provenance d'un appareil d'analyse de gaz dissous (DGA) (170) ;
au moins un processeur (255) configuré pour :

> - exécuter une analyse de flux de courant électrique du transformateur sur la base, au moins en partie, du premier courant de surveillance et du deuxième courant de surveillance ;
> - détecter, sur la base de l'analyse de flux de courant électrique, un état de défaut traversant ;
> - détecter, à partir des données DGA reçues, un état anormal lié au gaz ; et
- générer des données de santé du transformateur sur la base, au moins en partie, de l'état de défaut traversant et de l'état anormal lié au gaz ; et

une interface de sortie configurée pour transmettre à partir de l'appareil de diagnostic, au moins un parmi un signal lié à la santé du transformateur ou un signal de commande basé sur les données de santé du transformateur ;
dans lequel l'analyse de flux de courant comprend :

le calcul d'une valeur de courant électrique différentiel et d'une valeur de courant électrique de retenue sur la base du courant d'enroulement primaire et du courant d'enroulement secondaire du transformateur;
la déduction de données de flux de courant électrique en temps réel du transformateur en comparant un rapport entre la valeur de courant électrique différentiel et la valeur de courant électrique de retenue à une valeur seuil ;
l'identification d'un premier instant dans le temps où le rapport dépasse la valeur seuil ;
la récupération, à partir d'un élément de stockage (235), de données de flux de courant électrique historiques du transformateur ;
la génération de données de flux de courant électrique cumulatives du transformateur en combinant les données de flux de courant électrique historiques et les données de flux de courant électrique en temps réel obtenues au premier instant dans le temps et après le premier instant dans le temps ; et
la détection de l'état de défaut traversant sur la base des données de flux de courant électrique cumulatives du transformateur.

2. Appareil de diagnostic selon la revendication 1, comprenant en outre :
un relais de protection (260) configuré pour recevoir le signal de commande et modifier ou éliminer un flux de courant électrique à travers au moins un parmi un enroulement primaire du transformateur ou un enroulement secondaire du transformateur en activant un ou plusieurs éléments de protection couplés au transformateur.

3. Appareil de diagnostic selon la revendication 1, dans lequel au moins la première interface d'entrée (230), la deuxième interface d'entrée (225), la troisième interface d'entrée (215), l'interface de sortie, l'au moins un processeur (255), et l'élément de stockage (235) sont collectivement logés dans une enceinte (180).

4. Appareil de diagnostic selon la revendication 3, dans lequel l'enceinte comporte au moins un parmi un port de communication ou une interface utilisateur (275) qui permet à un utilisateur de définir la première valeur seuil.

5. Appareil de diagnostic selon la revendication 1, dans lequel le premier courant de surveillance est fourni à la première interface d'entrée par un premier transformateur de courant électrique couplé en série avec un enroulement primaire du transformateur (120) et le deuxième courant de surveillance est fourni à la deuxième interface d'entrée par un deuxième transformateur de courant électrique couplé en série avec un enroulement secondaire du transformateur (120).

6. Système de surveillance de santé de transformateur (100) comprenant :

au moins un élément de détection de courant électrique (165) couplé à un transformateur (120) ;
un premier disjoncteur (115, 125) couplé à l'un d'un enroulement primaire du transformateur ou d'un enroulement secondaire du transformateur;
un appareil d'analyse de gaz dissous (DGA) (170) couplé au transformateur ; et
un appareil de diagnostic (175) selon la revendication 1 couplé à l'au moins un élément de détection de courant électrique et à l'appareil DGA (170), et couplé en outre au premier disjoncteur, dans lequel l'appareil de diagnostic (175) est configuré pour transmettre un signal de commande pour activer le premier disjoncteur.

7. Système selon la revendication 6, dans lequel l'au moins un élément de détection de courant électrique comprend un premier élément de détection de courant électrique et un deuxième élément de détection de courant électrique, le premier élément de détection de courant électrique étant configuré pour détecter un courant d'enroulement primaire du transformateur et le deuxième élément de détection de courant électrique étant configuré pour détecter un courant d'enroulement secondaire du transformateur, et dans lequel l'analyse de flux de courant électrique comprend :

le calcul d'une valeur de courant électrique différentiel et d'une valeur de courant électrique de retenue sur la base du courant d'enroulement primaire et du courant d'enroulement secondaire du transformateur ;
la comparaison d'un rapport entre la valeur de courant électrique différentiel et la valeur de courant électrique de retenue à une valeur seuil ; et la déclaration de l'état de défaut traversant lorsque le rapport dépasse la valeur seuil.

8. Système selon la revendication 6, comprenant en outre au moins un parmi un moniteur de traversée, un moniteur de décharge partielle, ou un moniteur de changeur de prise en charge (OLTC) couplé à l'appareil de diagnostic (175), l'appareil de diagnostic étant en outre configuré pour générer des données de santé du transformateur sur la base, au moins en partie, de données d'événement reçues en provenance de l'au moins un parmi le moniteur de traversée, le moniteur de décharge partielle, ou le moniteur OLTC.

9. Système selon la revendication 6, dans lequel le signal de commande est configuré pour, au moins un parmi : activer un premier disjoncteur couplé à l'un d'un enroulement primaire du transformateur ou d'un enroulement secondaire du transformateur, ou activer un deuxième disjoncteur pour déconnecter au moins un premier alimenteur lorsqu'un état de défaut se produit dans le premier alimenteur, le premier alimenteur étant l'un d'une pluralité d'alimenteurs couplés à un enroulement secondaire du transformateur.

10. Système selon la revendication 6, comprenant en outre une pluralité d'alimenteurs (113, 114, 116) couplés à l'enroulement secondaire du transformateur, et comprenant en outre un deuxième disjoncteur (160), dans lequel l'appareil de diagnostic (175) est configuré pour transmettre un signal de commande afin d'activer le deuxième disjoncteur pour déconnecter un premier alimenteur (116) lorsqu'au moins un deuxième alimenteur parmi la pluralité d'alimenteurs est dans un état sans défaut lorsqu'un état de défaut traversant se produit dans le premier alimenteur (116).

11. Système selon la revendication 6, dans lequel le signal lié à la santé du transformateur est au moins un parmi un signal d'avertissement hors spécification qui indique que le transformateur n'est plus conforme à une ou plusieurs normes réglementaires, un signal d'avertissement de dégradation indiquant une durée de vie réduite du transformateur, ou un signal d'avertissement précoce indiquant au moins une parmi une cause ou une caractéristique d'une défaillance future du transformateur.

12. Système selon la revendication 6, dans lequel l'appareil de diagnostic (175) est en outre configuré pour :

   recevoir des données d'événement en provenance d'un relais de protection (260) ;
générer un enregistrement sur la base, au moins en partie, des données d'événement reçues en provenance du relais de protection (260) et des données DGA reçues en provenance de l'appareil DGA (170) ; et
délivrer en sortie, au moins une partie de l'enregistrement, via au moins une parmi une interface utilisateur (275) ou une liaison de communication de l'appareil de diagnostic (175).

13. Procédé comprenant :

   l'exécution dans un appareil de diagnostic (175) selon la revendication 1, d'un flux de courant électrique d'un transformateur (120), sur la base, au moins en partie, d'un premier courant de surveillance qui est proportionnel à un courant d'enroulement primaire du transformateur et d'un deuxième courant de surveillance qui est proportionnel à un courant d'enroulement secondaire du transformateur ;
   la détection, sur la base de l'analyse de flux de courant électrique, d'un état de défaut traversant ;
   la détection, à partir de données d'analyse de gaz dissous (DGA) reçues en provenance d'un appareil DGA (170), d'un état anormal lié au gaz dans le transformateur ;
   la génération de données de santé du transformateur sur la base, au moins en partie, de l'état de défaut traversant et de l'état anormal lié au gaz ; et
   la transmission d'au moins un parmi un signal lié à la santé du transformateur ou un signal de commande sur la base des données liées à la santé du transformateur;
   dans lequel l'exécution de l'analyse de flux de courant électrique comprend :

      le calcul d'une valeur de courant électrique différentiel et d'une valeur de courant électrique de retenue sur la base du courant d'enroulement primaire et du courant d'enroulement secondaire du transformateur;
      la déduction de données de flux de courant électrique en temps réel du transformateur en comparant un rapport entre la valeur de courant électrique différentiel et la valeur de courant électrique de retenue à une valeur seuil ;
      l'identification d'un premier instant dans le temps où le rapport dépasse la valeur seuil ;
      la récupération, à partir d'un élément de stockage (235), de données de flux de courant électrique historiques du transformateur ; et
      la génération de données de flux de courant électrique cumulatives du transformateur en combinant les données de flux de courant électrique historiques et les données de flux de courant électrique en temps réel obtenues au premier instant dans le temps et après le premier instant dans le temps ; et la détection de l'état de défaut traversant sur la base des données de flux de courant électrique cumulatives du transformateur.

14. Procédé selon la revendication 13, dans lequel les données de flux de courant électrique historiques sont horodatées, et dans lequel la récupération des données de flux de courant électrique historiques est basée, au moins en partie, sur une période de temps définissable avant le premier instant dans le temps.

15. Procédé selon la revendication 14, comprenant en outre :
la réception dans l'appareil de diagnostic (175), d'une entrée d'utilisateur comprenant au moins une parmi la valeur seuil ou une période de temps spécifiant la période de temps définissable.

16. Procédé selon la revendication 13, comprenant en outre :
l'affichage sur un afficheur de l'appareil de diagnostic (175), d'au moins un parmi un état de santé du transformateur, le premier instant dans le temps où le rapport a dépassé la valeur seuil, ou au moins une partie des données de flux de courant électrique cumulatives.

17. Procédé selon la revendication 13, dans lequel les données DGA reçues en provenance de l'appareil DGA (170) comprennent au moins une parmi des données DGA collectées par l'appareil DGA avant un premier instant dans le temps ou des données DGA collectées par l'appareil DGA après le premier instant dans le temps.

18. Procédé selon la revendication 17, dans lequel les données DGA collectées par l'appareil DGA (170) après le premier instant dans le temps indiquent que le transformateur présente au moins une parmi : une performance réduite, une performance compromise, une durée de vie réduite, ou une défaillance catastrophique à venir.

FIG. 1

FIG. 2

FIG. 3

400

```
┌─────────────────────────────────────────────────────────┐
│                                                    405    │
│  executing in a diagnostic apparatus, a current flow      │
│  analysis of a transformer, the current flow analysis     │
│  based at least in part on a first monitor current that   │
│  is proportional to a primary winding current of the      │
│  transformer and a second monitor current that is         │
│  proportional to a secondary winding current of the       │
│  transformer                                              │
└─────────────────────────────────────────────────────────┘
                            │
                            ▼
┌─────────────────────────────────────────────────────────┐
│                                                    410    │
│  detecting based on the current flow analysis, a          │
│  through fault condition                                  │
└─────────────────────────────────────────────────────────┘
                            │
                            ▼
┌─────────────────────────────────────────────────────────┐
│                                                    415    │
│  detecting, from dissolved gas analysis (DGA) data        │
│  received from a DGA apparatus, an abnormal gas-related    │
│  condition in the transformer                             │
└─────────────────────────────────────────────────────────┘
                            │
                            ▼
┌─────────────────────────────────────────────────────────┐
│                                                    420    │
│  generating transformer health data based at least in     │
│  part on the current flow analysis and the abnormal       │
│  gas-related condition                                    │
└─────────────────────────────────────────────────────────┘
                            │
                            ▼
┌─────────────────────────────────────────────────────────┐
│                                                    425    │
│  transmitting at least one of a transformer health        │
│  related signal or a control signal based on the          │
│  transformer health data                                  │
└─────────────────────────────────────────────────────────┘
```

# FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 70353315
- IN 201644003795
- US 2009043538 A
- US 2009091867 A
- WO 2013008246 A
- US 2011063761 A